# FASCICULE DE BREVET EUROPEEN

(11) **EP 1 506 284 B1**
(45) Date de publication et mention de la délivrance du brevet: **01.07.2009**
(21) Numéro de dépôt: 03752831.2
(22) Date de dépôt: 16.05.2003
(51) Int. Cl.: C12M 3/00, C12M 1/34

(54) **DISPOSITIF DE CULTURE DE CELLULES**
ZELLKULTURANLAGE
CELL CULTURE DEVICE

(30) Priorité: 21.05.2002 FR 0206186
(43) Date de publication de la demande: 16.02.2005
(73) Titulaire: INSTITUT NATIONAL DE LA RECHERCHE AGRONOMIQUE, 75007 Paris (FR)
(72) Inventeur: OZIL, Jean-Pierre, F-92370 Chaville (FR)
(74) Mandataire: Texier, Christian
(86) Numéro de dépôt international: PCT/FR2003/001499
(87) Numéro de publication internationale: WO 2003/097788

(56) Documents cités:
- WO-A-01/59447
- DE-A- 19 948 473

## Description

La présente invention concerne un dispositif de culture de cellules permettant leur traitement par différents milieux en évitant leur manipulation.

Le document FR 2659347 (publié le 13/09/1991) décrit un dispositif pour la culture de cellules comportant une enceinte destinée à recevoir les cellules. L'enceinte comprend une paroi horizontale formée par deux plaques de verre juxtaposées et espacées de manière à former entre elles une fente présentant une largeur inférieure aux diamètres des cellules. Les cellules à traiter sont positionnées sur la fente. Cette enceinte est destinée à contenir des milieux de culture ou d'impulsion. Les différents milieux sont injectés successivement dans l'enceinte par des tubulures distinctes positionnées au dessus de la paroi supportant les cellules. Ils sont par ailleurs évacués par une ou plusieurs tubulure(s) située(s) au-dessous de la paroi.

Dans ce dispositif, les cellules sont maintenues sur la fente grâce à la dépression provoquée par l'aspiration du milieu par la ou les tubulure(s) d'évacuation.

Un tel dispositif est destiné notamment à la culture d'ovocytes ou d'oeufs fécondés ou d'embryons.

En particulier, il peut être utilisé pour l'activation d'ovocytes expérimentaux, cette activation étant nécessaire pour le bon développement ultérieur des embryons. Pour provoquer cette activation, on injecte dans l'enceinte un milieu de culture et on positionne les cellules à activer dans ce milieu de culture. Le milieu de culture riche en ions est ensuite évacué et simultanément remplacé par un milieu d'impulsion contenant des ions Ca²⁺. Lorsque le milieu de culture a totalement été évacué et remplacé par le milieu d'impulsion, on soumet les cellules à une impulsion de champ électrique qui provoque l'électroperméabilisation transitoire de leur membrane plasmique et la pénétration des ions Ca²⁺ à l'intérieur des cellules. Puis le milieu d'impulsion est à son tour évacué et remplacé par le milieu de culture. Ces étapes sont répétées un certain nombre de fois de sorte que les cellules sont soumises à une série contrôlée d'impulsions calciques qui déclenche leur activation.

Un avantage de ce dispositif est qu'il permet le traitement des cellules par différents milieux en évitant leur manipulation.

Toutefois, un problème posé par ce type de dispositif est que le remplacement d'un milieu par un autre est relativement long, ce qui limite la fréquence d'alternance des milieux dans l'enceinte.

Par exemple, dans le cas où le dispositif est utilisé pour l'activation de cellules, un temps minimal d'injection du milieu d'impulsion est nécessaire (de l'ordre de 40 secondes) pour remplacer le milieu de culture par le milieu d'impulsion. Ce temps minimal garantit en effet un lavage suffisant des cellules par le milieu d'impulsion.

Dans le cas où ce temps minimal de lavage ne serait pas respecté, le milieu d'impulsion contiendrait des ions résiduels provenant du milieu de culture. Lors de l'application du champ électrique, ces ions induiraient un courant ionique trans-membranaire qui provoquerait la destruction des cellules.

Un autre problème lié au lavage est que l'exposition prolongée des cellules dans le milieu d'impulsion présentant une faible force ionique perturbe l'équilibre des cellules et les expose à des effets délétères. Pour préserver la survie cellulaire, il est donc nécessaire de réduire la durée de lavage des cellules.

Un but de l'invention est de fournir un dispositif de culture de cellules permettant de remplacer rapidement le milieu dans lequel les cellules sont placées.

On a remarqué que lors de l'injection du milieu d'impulsion, les ions présents dans les conduits destinés à l'injection du milieu de culture ont tendance à migrer vers l'enceinte et à contaminer le milieu d'impulsion. Ce phénomène augmente le temps de lavage nécessaire pour obtenir une baisse suffisante de la concentration ionique dans l'enceinte.

L'invention propose donc un dispositif destiné notamment à la culture de cellules comprenant une enceinte dans laquelle sont placées les cellules, ladite enceinte comportant au moins un premier conduit d'injection d'un premier type de fluide, au moins un deuxième conduit d'injection d'un deuxième type de fluide, caractérisé en ce qu'il comprend en outre un conduit de rétro-injection dont l'une des extrémités est connectée latéralement au premier conduit d'injection et l'autre extrémité est reliée à des moyens de pompage, les moyens de pompage étant aptes, lors de l'injection de fluide par le(s) deuxième(s) conduit(s) à aspirer le fluide contenu dans le premier conduit via le conduit de rétro-injection, de sorte que du fluide contenu dans le premier conduit ne diffuse pas dans le fluide injecté dans l'enceinte.

Dans ce dispositif, les moyens de pompages permettent d'inverser le flux de fluide dans le conduit d'injection non actif. Cette « rétro-injection » transitoire de fluide s'oppose à la diffusion passive des ions vers l'enceinte.

Cette caractéristique améliore considérablement la vitesse de remplacement d'un fluide par un autre dans l'enceinte et conduit à des temps de remplacement de l'ordre de la seconde.

Selon une mise en oeuvre préférée de l'invention, le dispositif comprend plusieurs conduits d'injection d'un même type de fluide et les embouchures desdits conduits d'injection sont disposés à intervalles réguliers le long de parois de l'enceinte.

Le dispositif peut comprendre plusieurs conduits d'injection de chaque fluide, chaque conduit d'injection du premier type de fluide étant superposé à un conduit d'injection du deuxième type de fluide.

Le dispositif peut comprendre plusieurs conduits d'injection de chaque fluide, chaque conduit d'injection du premier type de fluide étant positionné en face d'un conduit d'injection du deuxième type de fluide.

De préférence, le temps de remplacement d'un fluide par un autre dans l'enceinte est de l'ordre de la seconde.

Dans une mise en oeuvre de l'invention, le dispositif comprend des électrodes aptes à appliquer au fluide contenu dans l'enceinte un champ électrique maximum de l'ordre de 10 kV/cm.

L'invention propose également un procédé de culture de cellules comprenant les étapes selon lesquelles :
- les cellules sont placées en culture dans une enceinte,
- on injecte dans l'enceinte pendant un temps déterminé un premier type de fluide via au moins un premier conduit d'injection,
- on évacue le premier type de fluide et on injecte simultanément dans l'enceinte un deuxième type de fluide via au moins un deuxième conduit d'injection,
caractérisé en ce que durant l'étape d'injection du deuxième type de fluide, on aspire le fluide contenu dans le premier conduit de sorte que du fluide contenu dans le premier conduit ne diffuse pas dans le fluide injecté dans l'enceinte.

Selon une mise en oeuvre préférée de ce procédé, on aspire le fluide contenu dans le premier conduit via un conduit de rétro-injection dont l'une des extrémités est connectée latéralement au premier conduit d'injection et l'autre extrémité est reliée à des moyens de pompage.

Ce procédé peut en outre comprendre une étape selon laquelle on évacue le deuxième type de fluide et on injecte à nouveau dans l'enceinte le premier type de fluide via le premier conduit d'injection et on répète les étapes suivantes de sorte que les cellules baignent séquentiellement dans plusieurs types de fluide.

Selon une mise en oeuvre de ce procédé, les cellules sont soumises à un champ électrique durant l'injection de l'un des fluides.

D'autres caractéristiques et avantages ressortiront encore de la description qui suit, laquelle est purement illustrative et non limitative et doit être lue en regard des figures annexées parmi lesquelles :
- la figure 1 est un schéma représentatif d'un exemple de dispositif de culture de cellules conforme à une mise en oeuvre de l'invention,
- les figures 2 et 3 illustrent des étapes de fonctionnement du dispositif de la figure 1 dans le dans le cas où le dispositif est utilisé pour l'activation de cellules,
- la figure 4 est un schéma représentatif d'un exemple de dispositif de culture de cellules conforme à une autre mise en oeuvre de l'invention,
- la figure 5 est un schéma représentatif d'un exemple d'élément support pouvant être utilisé pour supporter les cellules à traiter dans une enceinte,
- la figure 6 est un schéma représentatif d'un autre exemple d'élément support pouvant être utilisé pour supporter les cellules à traiter dans une enceinte,
- la figure 7 est un schéma représentatif d'un exemple de dispositif permettant d'éviter la perturbation du traitement des cellules par des bulles de gaz.

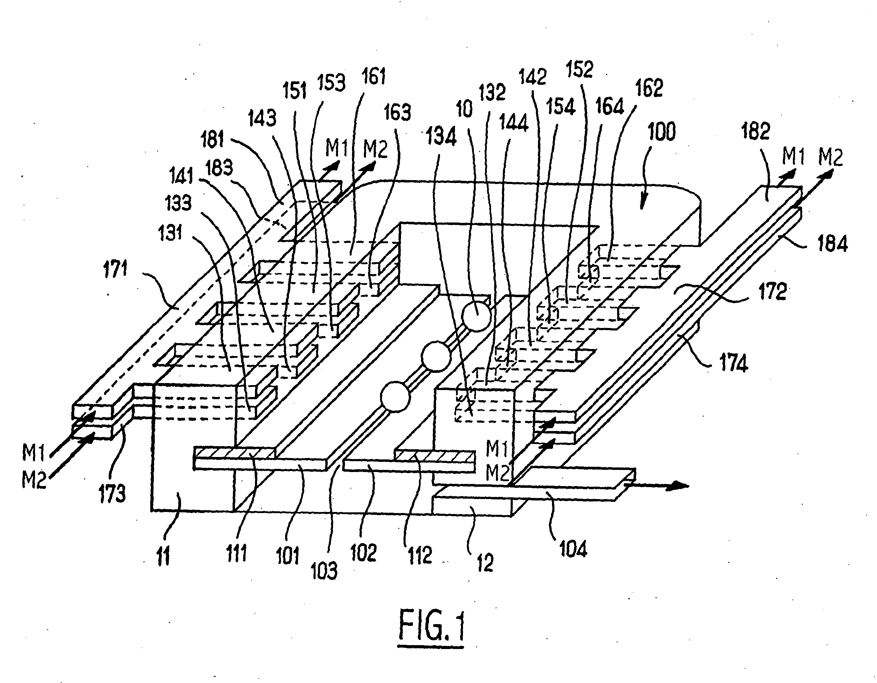

Sur la figure 1, le dispositif de culture de cellules représenté comprend une enceinte 100 comprenant des parois délimitant une chambre destinée à contenir un milieu fluide. Ce dispositif est dans cet exemple utilisé pour l'activation d'ovocytes. Dans la chambre, un élément support horizontal est positionné, formé par la juxtaposition de deux plaques de verre 101 et 102. Les plaques de verre 101 et 102 sont maintenues encastrées dans les parois 11 et 12 latérales de l'enceinte. Ces plaques de verre 101 et 102 sont espacées de manière à définir entre elles une fente rectiligne 103 de largeur inférieure au diamètre des ovocytes 10 à traiter.

L'enceinte 100 comprend également des électrodes 111 et 112 s'étendant longitudinalement de part et d'autre de la fente 103.

Un milieu de culture M1, riche en ions, peut être amené dans la partie supérieure de la chambre par deux ensembles de conduits parallèles 131, 141, 151, 161, et 132, 142, 152, 162 débouchant respectivement sur les parois latérales 11 et 12 de la chambre, positionnées de part et d'autre de la fente 103. Les embouchures des conduits 131, 141, 151, 161 font respectivement face aux embouchures des conduits 132, 142, 152, 162. Ces embouchures sont réparties régulièrement le long des parois 11 et 12 de l'enceinte 100 de sorte que le milieu M1 soit injecté sensiblement uniformément dans la partie supérieur de la chambre.

Un milieu d'impulsion M2 contenant des ions Ca²⁺ peut également être amené dans la partie supérieure de la chambre par deux ensembles de conduits parallèles 133, 143, 153, 163, et 134, 144, 154, 164 similaires aux ensembles de conduits d'amenée du milieu M1 et superposés à ceux-ci.

Le milieu contenu dans l'enceinte et correspondant à la phase de traitement en cours est évacué par un conduit d'évacuation 104 situé à un niveau inférieur à celui de l'élément support. Le courant de liquide maintient par dépression les ovocytes 10 plaqués sur la fente 103.

Les conduits parallèles 131, 141, 151, 161 sont reliés à un conduit transversal d'injection 171. Le conduit transversal 171 est connecté à l'une de ses extrémités à des moyens d'injection d'un fluide constitutif du milieu M1. Ce conduit 171 se prolonge à son autre extrémité en un conduit de rétro-injection 181 connecté à des moyens de pompage non représentés sur cette figure.

De manière symétrique, les conduits parallèles 132, 142, 152, 162 sont reliés à un conduit transversal d'injection 172. Le conduit transversal 172 est connecté à l'une de ses extrémités à des moyens d'injection de milieu M1. Ce conduit 172 se prolonge à son autre extrémité en un conduit de rétro-injection 182 connecté à des moyens de pompage.

De manière similaire, les conduits parallèles 133, 143, 153, 163 sont reliés à leur extrémité opposée à leur embouchure à un conduit transversal d'injection 173. Le conduit transversal 173 est connecté à l'une de ses extrémités à un réservoir contenant un fluide constitutif du milieu M2. Ce conduit 173 se prolonge à son autre extrémité en un conduit de rétro-injection 183 connecté également à des moyens de pompage.

Enfin, de manière symétrique, les conduits parallèles 134, 144, 154, 164 sont reliés à leur extrémité opposée à leur embouchure à un conduit transversal d'injection 174. Le conduit transversal 174 est connecté à l'une de ses extrémités au réservoir contenant le milieu M2. Ce conduit 174 se prolonge à son autre extrémité en un conduit de rétro-injection 184 connecté à des moyens de pompage.

Le milieu contenu dans la chambre, correspondant à la phase de traitement en cours, est évacué par un conduit 104 positionné à un niveau plus bas que celui des ovocytes 10. Le courant de fluide maintient par dépression les ovocytes plaqués sur la fente 103.

On va maintenant décrire les étapes d'un exemple de traitement pouvant être mis en oeuvre grâce à ce dispositif.

On considère que la chambre est préalablement remplie par du fluide correspondant au milieu de culture M1.

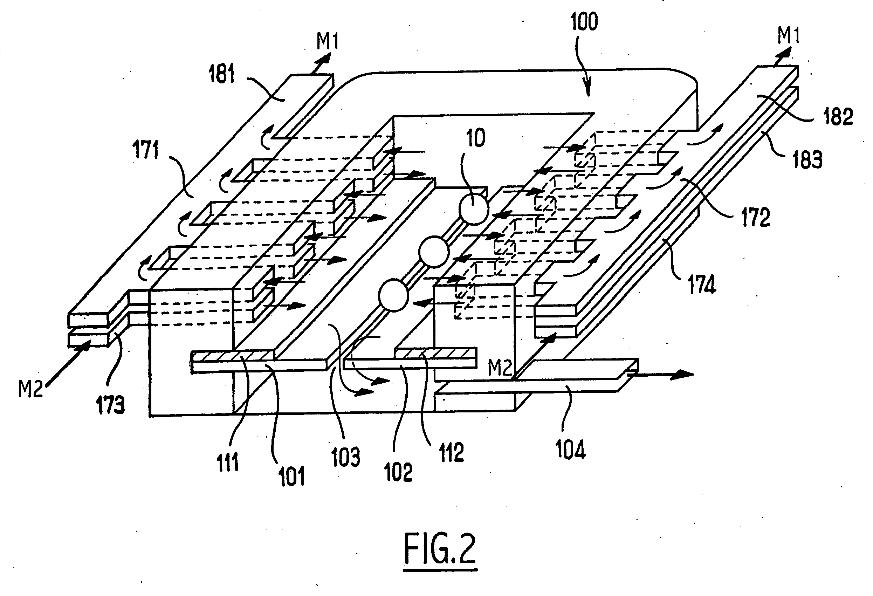

Sur la figure 2, on a représenté une première étape de traitement durant laquelle on remplace le milieu de culture M1 par le milieu d'impulsion M2. Selon cette étape, ainsi qu'indiqué par les flèches, on stoppe l'injection de milieu M1 par les conduits 171 et 172 et on injecte du milieu M2 par les conduits 173 et 174. Le milieu M1 est évacué par le conduit d'évacuation 104.

Les ions contenus dans le milieu de culture M1 qui remplit les conduits 131, 141, 151, 161 et 132, 142, 152, 162 ont naturellement tendance à migrer des zones à forte concentration ioniques vers les zones à faible concentration ionique, c'est à dire vers le fluide contenu dans la chambre. En effet, le fluide contenu dans la chambre s'appauvrit en ions à mesure que le milieu M1 est remplacé par le milieu M2, ce qui provoque la diffusion des ions du milieu M1 vers le milieu M2.

Pour contrer ce phénomène, on active les moyens de pompages connectés aux conduits 181 et 182 de manière à inverser le flux dans les conduits d'injection 131, 141, 151, 161 et 132,142, 152, 162.

Cette rétro-injection transitoire s'oppose à la diffusion des ions du milieu de culture M1 vers la chambre.

Pour un débit d'injection nominal dans la chambre d'un fluide non-ionique de 100 mL/heures, la diffusion ionique peut être stoppée en moins d'une seconde grâce à un flux de rétro-injection de 7,9 mL/heures, soit légèrement inférieur au débit nominal.

Le fait que les conduits d'injection soient répartis le long des parois latérales permet d'améliorer la vitesse de remplacement du milieu M1 par le milieu M2 en divers points de la chambre.

Après cette première étape de remplacement du milieu M1 par le milieu M2, les ovocytes 10 sont entièrement lavés par le milieu d'impulsion. Ils sont alors soumis à une impulsion de champ électrique pouvant atteindre 10 kV/cm. Le champ électrique entraîne l'électroperméabilisation de la membrane plasmique des ovocytes 10 et la pénétration des ions Ca²⁺ à l'intérieur de ceux-ci.

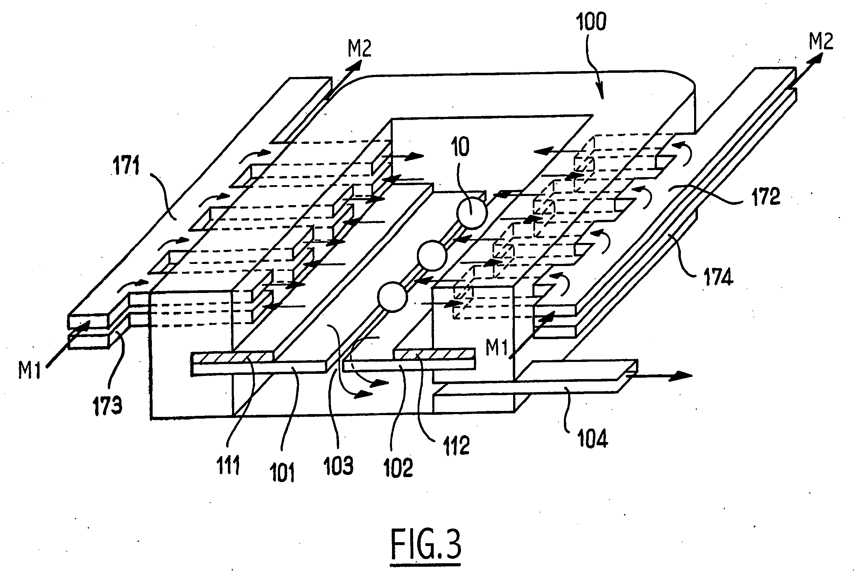

Sur la figure 3, on a représenté une seconde étape de traitement durant laquelle on remplace le milieu d'impulsion M2 par le milieu de culture M1. Selon cette étape, ainsi qu'indiqué par les flèches, on stoppe l'injection de milieu M2 par les conduits 173 et 174 et on injecte du milieu M1 par les conduits 171 et 172. Le milieu M2 est évacué par le conduit d'évacuation 104.

De manière similaire à la première étape, on peut activer les moyens de pompages connectés aux conduits 183 et 184 de manière à inverser le flux dans les conduits d'injection 133, 143, 153, 163 et 134, 144, 154, 164.

On a décrit l'utilisation du dispositif de la figure 1 dans le cadre de l'injection de deux types de fluides différents. On comprendra que ce type de dispositif peut être appliqué à l'injection de N fluides, cette injection pouvant être séquentielle ou simultanée.

Le réglage proportionnel des débits des fluides injectés permet de piloter dans la chambre les variations temporelles de concentrations simples ou complexes d'ions, de particules ou de molécules.

Ce dispositif permet également de régler avec précision la fréquence de ces variations. Dans le cas de l'activation d'ovocytes, la fréquence des impulsions calciques peut être réglée en fonction du type d'ovocytes à activer.

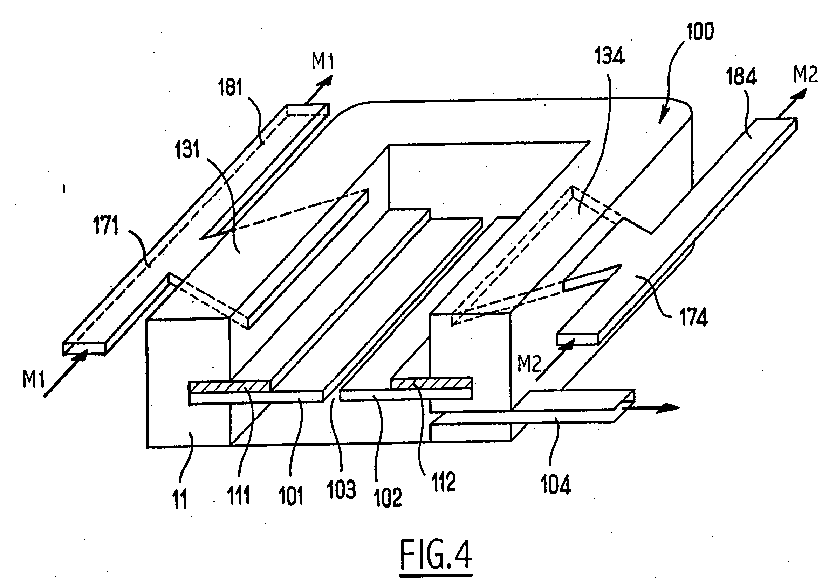

Sur la figure 4, le dispositif de culture représenté est similaire à celui des figures 1 à 3, excepté qu'il présente deux conduits d'injection 131 et 134, ces conduits étant destinés respectivement à l'injection des fluides M1 et M2. Chaque conduit d'injection 131, 134 présente une forme générale de triangle, dont le sommet est relié à un conduit transversal respectivement 171, 174 et dont la base forme une embouchure d'injection dans l'une des parois latérales 11 et 12 de la chambre.

Les embouchures des conduits d'injection 131 et 134 s'étendent le - long des parois 11 et 12 de l'enceinte 100 de sorte que les milieux M1 et M2 soient injectés sensiblement uniformément dans la partie supérieur de la chambre.

Cette mise en oeuvre est particulièrement avantageuse car elle permet de générer un flux d'injection quasi-laminaire, et par conséquent d'assurer un flux d'injection quasi-uniforme le long des parois de la chambre.

De manière indépendante, on a pu remarquer que les cellules avaient tendance à migrer sous l'effet des courants fluides générés par l'injection et l'évacuation du milieu contenu dans l'enceinte, cette migration pouvant notamment entraîner leur agglomération.

Dans le cas du dispositif représenté à la figure 1, les courants de fluide provoquent la translation des cellules, le long de la fente de l'élément support vers le centre de la chambre où elles de regroupent. Les cellules sont comprimées les unes sur les autres. La réduction de l'espace entre les cellules modifie l'efficacité du lavage à la périphérie de chaque cellule.

Pour pallier cet inconvénient, l'invention propose également un dispositif destiné notamment à la culture de cellules comprenant une enceinte dans laquelle sont placées les cellules, l'enceinte comprenant au moins un conduit d'injection de fluide et un conduit d'aspiration de fluide ainsi qu'un élément support comportant des orifices sur lesquels sont positionnées les cellules, caractérisé en ce qu'il comprend un élément support comportant des orifices destinés à recevoir des cellules, chaque orifice étant destiné à recevoir une cellule unique et présentant au moins une dimension minimale inférieure au diamètre de la cellule et au moins une dimension maximale supérieure à la dimension minimale de l'orifice, de sorte que l'orifice s'oppose au passage de la cellule à travers l'élément support tout en permettant le passage du fluide, et le fluide entrant dans l'enceinte est prélevé à travers les orifices en créant une dépression qui assure le blocage des cellules sur les orifices.

Le principe de ce dispositif peut être appliqué à tout type d'enceinte dans laquelle des cellules doivent être maintenues en place et dans laquelle un fluide circule.

Dans ce dispositif, chaque orifice reçoit une seule cellule. Cette caractéristique permet d'éviter la migration des cellules due aux courants fluides générés par l'injection et l'évacuation du milieu contenu dans l'enceinte.

En outre, ces orifices évitent que les cellules ne s'agglomèrent les unes avec les autres, ce qui garantit un bon lavage. En effet, le fluide de lavage circule sur toute la périphérie de la cellule pour être évacué à travers les orifices. Le lavage des cellules est efficace, ce qui réduit le temps de lavage nécessaire par rapport aux dispositifs de l'art antérieur.

De préférence, la dimension maximale d'un orifice est supérieure à 2 fois sa dimension minimale.

Par ailleurs, la dimension maximale d'un orifice est de préférence supérieure à 80µm.

En outre, la dimension minimale d'un orifice est de préférence inférieure à 40 µm.

Cette caractéristique permet d'éviter l'occultation d'une partie de la paroi cellulaire dans l'orifice. Ce qui permet de garantir un lavage efficace de la cellule par les milieux de traitement ou l'application d'un champ électrique sur la totalité de la paroi cellulaire.

Dans une mise en oeuvre de l'invention, les orifices présentent une forme oblongue.

Dans une autre mise en oeuvre de l'invention, les orifices présentent une forme générale de croix.

Typiquement, les orifices sont espacés d'un intervalle compris entre 120 et 240µm.

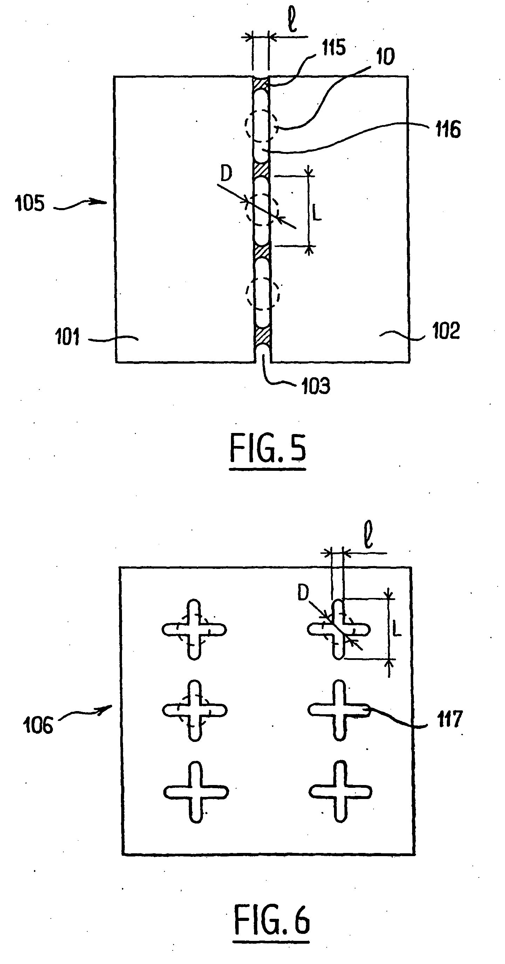

Sur la figure 5, on a représenté un premier type d'élément support 105. Cet élément support est formé par la juxtaposition de deux plaques de verre 101 et 102 destinées à être encastrées dans les parois latérales d'une enceinte ainsi que cela est représenté sur la figure 1. Ces plaques de verre 101 et 102 sont espacées de manière à définir entre elles une fente rectiligne 103 de largeur l=20µm.

Le diamètre des cellules à traiter varie en fonction du type de cellule. On considère que ces cellules présentent généralement un diamètre compris entre environ 80µm et 160µm. La largeur de la fente est donc toujours inférieure au diamètre D des cellules 10 à traiter quelque soit leur type (ces cellules sont représentées en traits pointillés).

Des joints 115 en polymères ont été disposés à intervalles réguliers dans la fente 103 pour former des logettes 116 sur lesquelles seront disposées les cellules 10. Les logettes 116 présentent une forme oblongue de longueur L=2×l=40µm.

Chaque logette 116 est destinée à recevoir une cellule 10 unique. La largeur l d'une logette étant inférieure au diamètre cellulaire, la cellule est maintenue en appui sur les plaques de verre 101 et 102. La longueur d'une logette étant supérieure à sa largeur I, la cellule n'occulte pas totalement la logette. Cette caractéristique permet le passage du flux de liquide de part et d'autre de la cellule. Le lavage de la cellule reste donc efficace.

En outre, le fait que les cellules 10 soient maintenues dans des logettes séparées empêche leur agglomération.

Dans une variante, l'élément support de la figure 5 est formé d'une plaque de verre unique et les logettes 116 sont des orifices de géométrie oblongue percés dans la plaque de verre.

Les logettes sont réparties sur la plaque de verre à intervalles réguliers, de préférence compris entre 120 et 240µm.

Sur la figure 6, on a représenté un deuxième type d'élément support 106. Cet élément support est formé d'une plaque de verre unique dans laquelle des logettes 117 ont été formées. Les logettes 117 sont des orifices en forme générale de croix. La largeur l des bras de la croix est inférieure au diamètre cellulaire D et la longueur L de chaque bras de la croix est supérieure à la largeur l.

Bien entendu, d'autres formes de logettes peuvent être réalisées. Pour maintenir la cellule en place sur l'élément support, ces logettes présentent au moins une dimension l inférieure au diamètre de la cellule: Pour garantir une circulation de fluide autour des cellules, ces logettes présentent au moins une dimension L supérieure à la largeur l.

Toutefois, la dimension l ne doit pas être trop proche du diamètre cellulaire le plus petit Par exemple, la dimension l sera de préférence inférieure à 40µm pour assurer un maintien de la cellule au-dessus du niveau de l'élément support. En effet, dans le cas où la dimension l serait trop proche du diamètre cellulaire, la dépression créée par le courant fluide passant à travers les logettes provoquerait l'aspiration partielle des cellules de petit diamètre à travers les logettes. Ce qui aurait pour conséquence d'occulter une portion de la paroi de ces cellules. Cette portion occultée de la paroi cellulaire ne serait donc plus soumise aux lavages par les milieux de traitement ou à l'application d'un champ électrique. L'efficacité du traitement appliqué s'en trouverait réduite.

De manière également indépendante, on a remarqué la formation de bulles de gaz sur les parois des circuits hydrauliques des dispositifs de culture de cellule. Ces bulles naissent spontanément dans les conduits et sont entraînée par le courant de fluide. Elles percutent et déplacent les cellules de leur fixation.

En particulier, dans le cas du dispositif de culture de la figure 1, les cellules sont entraînées et se superposent entre les électrodes. Elles peuvent créer des ponts de conductivité entre les électrodes au moment des impulsions électriques. Elles peuvent être parfois être évacuées avec le flux de liquide en dehors de la chambre.

La perturbation du traitement des cellules par les bulles condamne définitivement le succès du traitement.

Pour pallier cet inconvénient, l'invention propose un dispositif destiné notamment à la culture de cellules comprenant une enceinte dans laquelle sont placées les cellules, l'enceinte comprenant au moins un conduit d'injection de fluide et un conduit d'aspiration de fluide, caractérisé en ce qu'il comprend en outre une grille positionnée devant des embouchures des conduits d'injection.

Cette grille peut être utilisée dans tout type d'enceinte dans laquelle des cellules doivent être maintenues en place et dans laquelle un fluide circule.

Dans ce dispositif, la grille permet de collecter les bulles à la sortie des conduits d'injection. Les bulles, guidées par la grille, ont alors tendance à s'agglomérer à la surface du fluide de la chambre. Les cellules restent donc en place.

Typiquement, la grille présente un maillage dont les motifs présentent des dimensions de l'ordre de 100µm.

De manière préférentielle, la grille est placée à une distance de l'ordre de quelques dixièmes de millimètres à 1 millimètre des embouchures des conduits d'injection.

De manière préférentielle également, la grille est inclinée d'un angle sensiblement égal à 70 degrés par rapport à l'horizontale.

Dans une mise en oeuvre de l'invention, la grille comprend des déflecteurs latéraux au dessus de la surface libre du fluide pour empêcher des bulles en surface du fluide de contourner la grille.

De préférence, la ou les grille(s) s'étend(ent) devant chaque embouchure d'injection de fluide.

Par ailleurs, les embouchures des conduits étant réparties sur deux parois latérales opposées de l'enceinte, le dispositif peut comprendre deux grilles s'étendant chacune en face de l'une des parois latérales.

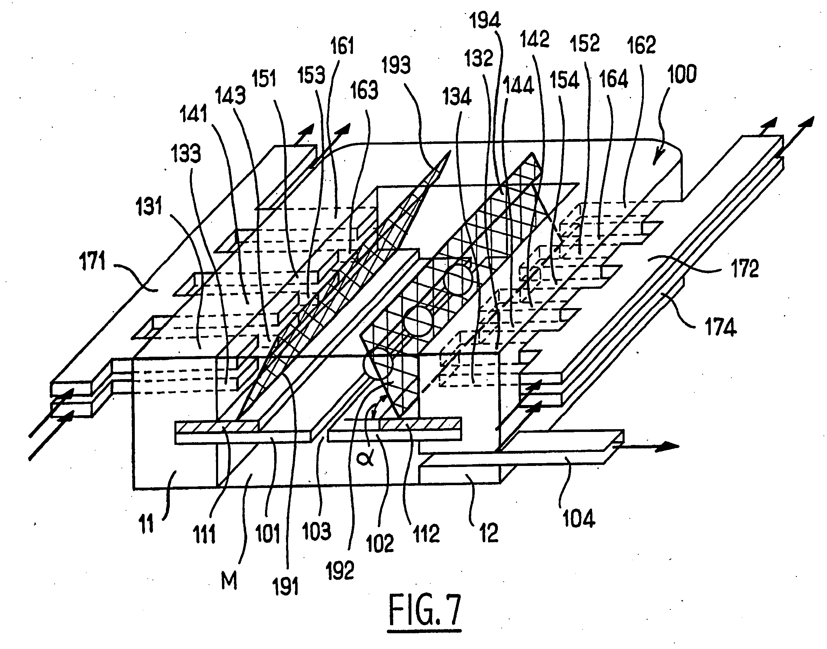

Sur la figure 7, on a représenté un dispositif similaire à celui de la figure 1 sur lequel deux grilles 191 et 192 ont été positionnées symétriquement en face respectivement des embouchures des ensembles de conduits d'injection 131, 133, 141, 143, 151, 153, 161, 163 et 132, 134, 142, 144, 152, 154, 162, 164 pour collecter les bulles. Le maillage de chaque grille 191, 192 présente des dimensions de l'ordre de 100µm × 100µm. Les grilles 191, 192 présentant un tel maillage dévient les bulles émergeant des conduits d'injection sans altérer le flux de fluide injecté.

La partie inférieure de la grille 191 est positionnée à environ 1 mm de distance des embouchures des conduits inférieurs 133, 143, 153, 163. En outre, elle est inclinée d'un angle a d'environ 70 degrés par rapport à l'horizontale.

De même, la partie inférieure de la grille 192 est positionnée à environ 1 mm de distance des embouchures des conduits inférieurs 134, 144, 154, 164. Elle est également inclinée d'un angle a d'environ 70 degrés par rapport à l'horizontale.

L'inclinaison des grilles oriente le flux des bulles vers la surface libre du fluide M contenu dans l'enceinte.

Certaines bulles crèvent en arrivant à la surface du fluide.

D'autres, plus stables, s'agglomèrent. Ces bulles peuvent fusionner pour former des bulles plus grosses.

Les tensions superficielles permettent aux bulles de s'élever au-dessus de la surface libre du fluide.

Les grilles 191, 192 comprennent en outre des déflecteurs latéraux 193 et 194 au-dessus de la surface libre du fluide M. Ces déflecteurs évitent que des bulles n'empruntent les ponts de fluide créés par les forces de tension superficielle aux abords des parois de l'enceinte 100 pour rejoindre la partie centrale de la chambre où les cellules 10 sont positionnées.

Ces grilles peuvent être réalisées en métal ou en plastique.

## Revendications

1. Dispositif destiné à la culture de cellules (10) comprenant une enceinte (100) destinée à recevoir les cellules (10), ladite enceinte (100) comportant au moins un premier conduit d'injection (131, 141, 151, 161, 132, 142, 152, 162) d'un premier type de fluide (M1), au moins un deuxième conduit d'injection (133, 143, 153, 163, 134, 144, 154, 164) d'un deuxième type de fluide (M2), **caractérisé en ce qu'**il comprend en outre un conduit de rétro-injection (181, 182) dont l'une des extrémités est connectée latéralement au premier conduit d'injection (131, 141, 151, 161, 132, 142, 152, 162) et l'autre extrémité est reliée à des moyens de pompage, les moyens de pompage étant aptes, lors de l'injection de fluide par le(s) deuxième(s) conduit(s) (133, 143, 153, 163, 134, 144, 154, 164) à aspirer le fluide contenu dans le premier conduit via le conduit de rétro-injection (181, 182), de sorte que du fluide (M1) contenu dans le premier conduit ne diffuse pas dans le fluide (M2) injecté dans l'enceinte (100).

2. Dispositif selon la revendication 1, **caractérisé en ce qu'**il comprend plusieurs conduits d'injection d'un même type de fluide et **en ce que** les embouchures desdits conduits d'injection (131, 141, 151, 161, 132, 142, 152, 162 ; 133, 143, 153, 163, 134, 144, 154, 164) sont disposés à intervalles réguliers le long de l'une des parois (11, 12) de l'enceinte (100).

3. Dispositif selon la revendication 1, **caractérisé en ce qu'**il comprend un conduit d'injection (131, 134) dont l'embouchure s'étend le long de l'une des parois (11, 12) de l'enceinte (100).

4. Dispositif selon l'une des revendications qui précèdent, **caractérisé en ce qu'**il comprend un conduit d'injection (131, 134) présentant une forme générale évasée vers l'enceinte (100).

5. Dispositif selon l'une des revendications qui précèdent, **caractérisé en ce qu'**il comprend plusieurs conduits d'injection (131, 141, 151, 161, 132,142,152,162,133,143,153,163,134,144,154,164) dont les embouchures sont disposées les unes en face des autres sur deux parois (11, 12) opposées de l'enceinte (100).

6. Dispositif selon l'une des revendications qui précèdent, **caractérisé en** le remplacement d'un fluide par un autre dans l'enceinte est de l'ordre de la seconde.

7. Dispositif selon l'une des revendications qui précèdent, **caractérisé en ce qu'**il comprend des électrodes (111, 112) aptes à appliquer au fluide contenu dans l'enceinte un champ électrique maximal de l'ordre de 10 kV/cm.

8. Dispositif selon l'une des revendications qui précèdent, **caractérisé en ce qu'**il comprend un élément support (105, 106) comportant des orifices (116, 117) destinés à recevoir des cellules (10), et **en ce que** chaque orifice (116, 117) est destiné à recevoir une cellule (10) unique et présente au moins une dimension minimale (l) inférieure au diamètre (D) de la cellule (10) et au moins une dimension maximale (L) supérieure à la dimension minimale (l) de l'orifice de sorte que l'orifice (116, 117) s'oppose au passage de la cellule (10) à travers l'élément support (105, 106) tout en permettant le passage du fluide, et le fluide entrant dans l'enceinte (100) est prélevé à travers les orifices (116, 117) en créant une dépression qui assure le blocage des cellules (10) sur les orifices.

9. Dispositif selon la revendication 8, **caractérisé en ce que** la dimension maximale (L) d'un orifice (116, 117) est supérieure à 2 fois sa dimension minimale (l).

10. Dispositif selon l'une des revendications 8 ou 9, **caractérisé en ce que** la dimension maximale (L) d'un orifice est supérieure à 80µm.

11. Dispositif selon l'une des revendications 8 à 10, **caractérisé en ce que** la dimension minimale (l) d'un orifice est inférieure à 40µm.

12. Dispositif selon l'une des revendication 8 à 11, **caractérisé en ce que** les orifices (116, 117) présentent une forme oblongue.

13. Dispositif selon l'une des revendications 8 à 11, **caractérisé en ce que** les orifices (116, 117) présentent une forme générale de croix.

14. Dispositif selon l'une des revendications 8 à 13, **caractérisé en ce que** les orifices sont espacés d'un intervalle compris entre 120 et 240µm.

15. Dispositif selon l'une des revendications qui précédent, **caractérisé en ce qu'**il comprend une grille (191, 192) positionnée devant des embouchures des conduits d'injection (131, 141, 151, 161, 133, 143, 153, 163 ; 132, 142, 152, 162, 134, 144, 154, 164).

16. Dispositif selon la revendication 15, **caractérisé en ce que** la grille présente un maillage dont les motifs présentent des dimensions de l'ordre de 100µm.

17. Dispositif selon l'une des revendications 15 ou 16, **caractérisé en ce que** la grille (191, 192) est placée à une distance comprise entre 0,5 et 1 mm des embouchures des conduits d'injection (131, 141, 151, 161, 133, 143, 153, 163 ; 132, 142, 152, 162, 134, 144, 154, 164).

18. Dispositif selon l'une des revendications 15 à 17, **caractérisé en ce que** la grille (191, 192) est inclinée d'un angle sensiblement égal à 70 degrés par rapport à l'horizontale.

19. Dispositif selon l'une des revendications 15 à 18, **caractérisé en ce que** la grille (191, 192) comprend des déflecteurs latéraux (193, 194) au dessus de la surface libre du fluide pour empêcher des bulles en surface du fluide (M) de contourner la grille (191, 192).

20. Dispositif selon l'une des revendications 15 à 19, **caractérisé en ce que** la ou les grille(s) s'étend(ent) devant chaque embouchure d'injection de fluide (131, 141, 151, 161; 133, 143, 153, 163 ; 132, 142, 152, 162, 134, 144, 154, 164).

21. Dispositif selon la revendication 20, **caractérisé en ce que** les embouchures des conduits (131, 141, 151, 161, 133, 143, 153, 163 ; 132, 142, 152, 162, 134, 144, 154, 164) étant réparties sur deux parois (11, 12) latérales opposées de l'enceinte (100), il comprend deux grilles (191, 192) s'étendant chacune en face de l'une des parois latérales (11, 12).

22. Dispositif selon l'une des revendications 15 à 21, **caractérisé en ce que** la ou les grille(s) sont en métal ou en plastique.

23. Procédé de culture de cellules (10) selon lequel :
- les cellules (10) sont placées en culture dans une enceinte (100),
- on injecte dans l'enceinte (100) pendant un temps déterminé un premier type de fluide (M1) via au moins un premier conduit d'injection (131, 141, 151, 161, 132, 142, 152, 162),
- on évacue le premier type de fluide (M1) et on injecte simultanément dans l'enceinte (100) un deuxième type de fluide (M2) via au moins un deuxième conduit d'injection (133, 143, 153, 163, 134, 144, 154, 164),
**caractérisé en ce que** durant l'étape d'injection du deuxième type de fluide (M2), on aspire le fluide (M1) contenu dans le premier conduit (131, 141, 151, 161, 132, 142, 152, 162) de sorte que du fluide (M1) contenu dans le premier conduit (131, 141, 151, 161, 132, 142, 152, 162) ne diffuse pas dans le fluide (M2) injecté dans l'enceinte (100).

24. Procédé selon la revendication 23, **caractérisé en ce qu'**on aspire le fluide (M1) contenu dans le premier conduit via un conduit de rétro-injection (181, 182) dont l'une des extrémités est connectée latéralement au premier conduit d'injection (131, 141, 151, 161, 133, 143, 153, 163) et l'autre extrémité est reliée à des moyens de pompage.

25. Procédé selon l'une des revendications 23 ou 24, **caractérisé en ce qu'**il comprend en outre une étape selon laquelle on évacue le deuxième type de fluide (M2) et on injecte à nouveau dans l'enceinte (100) le premier type de fluide (M1) via le premier conduit d'injection (131, 141, 151, 161, 132, 142, 152, 162) et **en ce qu'**on répète les étapes suivantes de sorte que les cellules (10) baignent séquentiellement dans plusieurs types de fluide.

26. Procédé selon l'une des revendications 23 à 25, **caractérisé en ce que** durant l'injection de l'un des fluides, les cellules (10) sont soumises à un champ électrique.

27. Procédé selon l'une des revendications qui précèdent **caractérisé en ce que** l'étape de remplacement d'un fluide par un autre dans l'enceinte dure environ 1 seconde.

## Claims

1. A device for culturing cells (10) comprising an enclosure (100) for receiving the cells (10), said enclosure (100) having at least a first duct (131, 141, 151, 161, 132, 142, 152, 162) for injecting a first type of fluid (M1), at least a second duct (133, 143, 153, 163, 134, 144, 154, 164) for injecting a second type of fluid (M2), **characterized in that** it further comprises a back-injection duct (181, 182) one end of which is laterally connected to the first injection duct (131, 141, 151, 161, 132, 142, 152, 162), and the other end is linked to pumping means, the pumping means being capable, during fluid injection by the second duct(s) (133, 143, 153, 163, 134, 144, 154, 164) of drawing the fluid contained in the first duct via the back-injection duct (181, 182), so that the fluid (M1) contained in the first duct does not diffuse into the fluid (M2) injected into the enclosure (100).

2. The device according to claim 1, **characterized in that** it comprises several ducts for injecting the same type of fluid, and that the openings of said injection ducts (131, 141, 151, 161, 132, 142, 152, 162; 133, 143, 153, 163, 134, 144, 154, 164) are arranged at regular intervals along one of the walls (11, 12) of the enclosure (100).

3. The device according to claim 1, **characterized in that** it comprises an injection duct (131, 134) the opening of which extends along one of the walls (11, 12) of the enclosure (100).

4. The device according to any of the preceding claims, **characterized in that** it comprises an injection duct (131, 134) having a general shape flared towards the enclosure (100).

5. The device according to any of the preceding claims, **characterized in that** it comprises several injection ducts (131, 141, 151, 161, 132, 142, 152, 162, 133, 143, 153, 163, 134, 144, 154, 164) the openings of which are arranged facing each other on two opposite walls (11, 12) of the enclosure (100).

6. The device according to any of the preceding claims, **characterized in that** the replacement of one fluid with another inside the enclosure takes roughly one second.

7. The device according to any of the preceding claims, **characterized in that** it comprises electrodes (111, 112) adapted for applying to the fluid contained in the enclosure a maximum electrical field of roughly 10 kV/cm.

8. The device according to any of the preceding claims, **characterized in that** it comprises a supporting member (105, 106) having orifices (116, 117) for receiving cells (10), and **in that** each orifice (116, 117) is designed for receiving a single cell (10) and has at least a minimum dimension (I) smaller than the diameter (D) of the cell (10) and at least a maximum dimension (L) greater than the minimum dimension (I) of the orifice, so that the orifice (116, 117) is opposed to letting the cell (10) go through the supporting member (105, 106) while letting the fluid pass, and the fluid entering into the enclosure (100) is tapped through the orifices (116, 117) creating a negative pressure which ensures that the cells (10) are blocked at the orifices.

9. The device according to claim 8, **characterized in that** the maximum dimension (L) of an orifice (116, 117) is greater than twice the minimum dimension (I) thereof.

10. The device according to any of claims 8 or 9, **characterized in that** the maximum dimension (L) of an orifice is greater than 80 µm.

11. The device according to any of claims 8 to 10, **characterized in that** the minimum dimension (I) of an orifice is smaller than 40 µm.

12. The device according to any of claims 8 to 11, **characterized in that** the orifices (116, 117) have an elongated shape.

13. The device according to any of claims 8 to 11, **characterized in that** the orifices (116, 117) are generally cross-shaped.

14. The device according to any of claims 8 to 13, **characterized in that** the orifices are spaced by an interval between 120 and 240 µm.

15. The device according to any of the preceding claims, **characterized in that** it comprises a grid (191, 192) positioned in front of the openings of the injection ducts (131, 141, 151, 161, 133, 143, 153, 163; 132, 142, 152, 162, 134, 144, 154, 164).

16. The device according to claim 15, **characterized in that** the grid has a lattice structure the patterns of which have dimensions of roughly 100 µm.

17. The device according to any of claims 15 or 16, **characterized in that** the grid (191, 192) is placed at a distance between 0.5 and 1 mm of the openings of the injection ducts (131, 141, 151, 161, 133, 143, 153, 163; 132, 142, 152, 162, 134, 144, 154, 164).

18. The device according to any of claims 15 to 17, **characterized in that** the grid (191, 192) is sloped at an angle substantially equal to 70 degrees with respect to the horizontal.

19. The device according to any of claims 15 to 18, **characterized in that** the grid (191, 192) comprises lateral deflectors (193, 194) above the free area of the fluid in order to prevent surface bubbles of the fluid (M) from bypassing the grid (191, 192).

20. The device according to any of claims 15 to 19, **characterized in that** the grid(s) extend(s) in front of each fluid injection opening (131, 141, 151, 161, 133, 143, 153, 163; 132, 142, 152, 162, 134, 144, 154, 164).

21. The device according to claim 20, **characterized in that** as the openings of the ducts (131, 141, 151, 161, 133, 143, 153, 163; 132, 142, 152, 162, 134, 144, 154, 164) are distributed over the two opposite side walls (11, 12) of the enclosure (100), it comprises two grids (191, 192) each extending while facing one of the side walls (11, 12).

22. The device according to any of claims 15 to 21, **characterized in that** the grid(s) are composed of metal or plastic.

23. A method for culturing cells (10), according to which:
- the cells (10) are placed in a culture inside an enclosure (100),
- for a given period of time, a first type of fluid (M1) is injected into the enclosure (100) via at least a first injection duct (131, 141, 151, 161, 132, 142, 152, 162),
- the first type of fluid (M1) is discharged, and simultaneously, a second type of fluid (M2) is injected into the enclosure via at least a second injection duct (133, 143, 153, 163, 134, 144, 154, 164),
**characterized in that** during the step of injecting the second type of fluid (M2), the fluid (M1) contained in the first duct (131, 141, 151, 161, 132, 142, 152, 162) is aspirated so that the fluid (M1) contained in the first duct (131, 141, 151, 161, 132, 142, 152, 162) does not diffuse into the fluid (M2) injected into the enclosure (100).

24. The method according to claim 23, **characterized in that** the fluid (M1) contained in the first duct is sucked in via a back-injection duct (181, 182) having one the ends thereof laterally connected to the first injection duct (131, 141, 151, 161, 133, 143, 153, 163), and the other end is linked to pumping means.

25. The method according to any of claims 23 or 24, **characterized in that** it further comprises a step in which the second type of fluid (M2) is discharged, and the first type of fluid (M1) is again injected into the enclosure (100) via the first injection duct (131, 141, 151, 161, 132, 142, 152, 162), and that the following steps are repeated so that the cells (10) are sequentially bathing in several types of fluids.

26. The method according to any of claims 23 to 25, **characterized in that** during the injection of one of the fluids, the cells (10) are subjected to an electrical field.

27. The method according to any of the preceding claims, **characterized in that** the step of replacing one fluid with another in the enclosure takes about 1 second.

## Patentansprüche

1. Vorrichtung zur Zellkultur (10) umfassend einen Behälter (100) zum Aufnehmen der Zellen (10), wobei der Behälter (100) mindestens eine erste Leitung (131, 141, 151, 161, 132, 142, 152, 162) zum Injizieren einer ersten Fluidart (M1), mindestens eine zweite Leitung (133, 143, 153, 163, 134, 144, 154, 164) zum Injizieren einer zweiten Fluidart (M2) aufweist, **dadurch gekennzeichnet, dass** sie ferner eine Rückinjektionsleitung (181, 182) umfasst, deren eines Ende seitlich an die erste Injektionsleitung (131, 141, 151, 161, 132, 142, 152, 162) angeschlossen ist, und deren anderes Ende mit Pumpmitteln verbunden ist, wobei die Pumpmittel in der Lage sind, während der Fluidinjektion durch die zweite(n) Leitung(en) (133, 143, 153, 163, 134, 144, 154, 164) das in der ersten Leitung enthaltene Fluid über die Rückinjektionsleitung (181, 182) anzusaugen, so dass sich das in der ersten Leitung enthaltene Fluid (M1) nicht in das Fluid (M2), das in den Behälter (100) injiziert wird, ausbreitet.

2. Vorrichtung nach Anspruch 1, **dadurch gekennzeichnet, dass** sie mehrere Leitungen zum Injizieren derselben Fluidart umfasst, und dass die Mündungen der Injektionsleitungen (131, 141, 151, 161, 132, 142, 152, 162; 133, 143, 153, 163, 134, 144, 154, 164) in regelmäßigen Abständen an einer der Wände (11, 12) des Behälters (100) entlang angeordnet sind.

3. Vorrichtung nach Anspruch 1, **dadurch gekennzeichnet, dass** sie eine Injektionsleitung (131, 134) umfasst, deren Mündung sich an einer der Wände (11, 12) des Behälters (100) entlang erstreckt.

4. Vorrichtung nach einem der vorhergehenden Ansprüche, **dadurch gekennzeichnet, dass** sie eine Injektionsleitung (131, 134) umfasst, die eine allgemeine Form aufweist, die zum Behälter (100) hin erweitert ist.

5. Vorrichtung nach einem der vorhergehenden Ansprüche, **dadurch gekennzeichnet, dass** sie mehrere Injektionsleitungen (131, 141, 151, 161, 132, 142, 152, 162, 133, 143, 153, 163, 134, 144, 154, 164) umfasst, deren Mündungen einander gegenüber auf zwei entgegengesetzten Wänden (11, 12) des Behälters (100) angeordnet sind.

6. Vorrichtung nach einem der vorhergehenden Ansprüche, **dadurch gekennzeichnet, dass** das Ersetzen eines Fluids durch ein anderes in dem Behälter ungefähr eine Sekunde dauert.

7. Vorrichtung nach einem der vorhergehenden Ansprüche, **dadurch gekennzeichnet, dass** sie Elektroden (111, 112) umfasst, die dazu geeignet sind, auf das in dem Behälter enthaltene Fluid ein maximales elektrisches Feld von ungefähr 10 kV/cm anzuwenden.

8. Vorrichtung nach einem der vorhergehenden Ansprüche, **dadurch gekennzeichnet, dass** sie ein Stützelement (105, 106) umfasst, das Öffnungen (116, 117) zum Aufnehmen der Zellen (10) umfasst, und dass jede Öffnung (116, 117) dazu ausgelegt ist, eine einzelne Zelle (10) aufzunehmen und mindestens eine Mindestabmessung (I), die kleiner ist als der Durchmesser (D) der Zelle (10), und mindestens eine Höchstabmessung (L), die größer ist als die Mindestabmessung (I) der Öffnung, aufweist, so dass die Öffnung (116, 117) sich dem Durchgang der Zelle (10) durch das Stützelement (105, 106) entgegenstellt, während sie den Durchgang des Fluids zulässt, und das Fluid, das in den Behälter (100) eindringt, durch die Öffnungen (116, 117) hindurch abgenommen wird, wobei ein Unterdruck aufgebaut wird, der sicherstellt, dass die Zellen (10) an den Öffnungen blockiert werden.

9. Vorrichtung nach Anspruch 8, **dadurch gekennzeichnet, dass** die Höchstabmessung (L) einer Öffnung (116, 117) mehr als zweimal so groß wie ihre Mindestabmessung (I) ist.

10. Vorrichtung nach einem der Ansprüche 8 oder 9, **dadurch gekennzeichnet, dass** die Höchstabmessung (L) einer Öffnung größer ist als 80 µm.

11. Vorrichtung nach einem der Ansprüche 8 bis 10, **dadurch gekennzeichnet, dass** die Mindestabmessung (I) einer Öffnung kleiner ist als 40 µm.

12. Vorrichtung nach einem der Ansprüche 8 bis 11, **dadurch gekennzeichnet, dass** die Öffnungen (116, 117) eine längliche Form haben.

13. Vorrichtung nach einem der Ansprüche 8 bis 11, **dadurch gekennzeichnet, dass** die Öffnungen (116, 117) allgemein kreuzförmig sind.

14. Vorrichtung nach einem der Ansprüche 8 bis 13, **dadurch gekennzeichnet, dass** die Öffnungen um einen Abstand zwischen 120 und 240 µm beabstandet sind.

15. Vorrichtung nach einem der vorhergehenden Ansprüche, **dadurch gekennzeichnet, dass** sie ein Gitter (191, 192) umfasst, das vor den Mündungen der Injektionsleitungen (131, 141, 151, 161, 133, 143, 153, 163; 132, 142, 152, 162, 134, 144, 154, 164) angeordnet ist.

16. Vorrichtung nach Anspruch 15, **dadurch gekennzeichnet, dass** das Gitter eine Netzstruktur aufweist, deren Muster Abmessungen von ungefähr 100 µm aufweisen.

17. Vorrichtung nach einem der Ansprüche 15 oder 16, **dadurch gekennzeichnet, dass** das Gitter (191, 192) in einem Abstand zwischen 0,5 und 1 mm von den Mündungen der Injektionsleitungen (131, 141, 151, 161, 133, 143, 153, 163; 132, 142, 152, 162, 134, 144, 154, 164) angeordnet ist.

18. Vorrichtung nach einem der Ansprüche 15 bis 17, **dadurch gekennzeichnet, dass** das Gitter (191, 192) um einen Winkel geneigt ist, der im Wesentlichen gleich 70 Grad gegenüber der Waagerechten ist.

19. Vorrichtung nach einem der Ansprüche 15 bis 18, **dadurch gekennzeichnet, dass** das Gitter (191, 192) seitliche Ablenker (193, 194) über der freien Fläche des Fluids umfasst, um Oberflächenblasen des Fluids (M) daran zu hindern, an dem Gitter (191, 192) vorbeizukommen.

20. Vorrichtung nach einem der Ansprüche 15 bis 19, **dadurch gekennzeichnet, dass** das bzw. die Gitter sich vor jeder Fluidinjektionsmündung (131, 141, 151, 161, 133, 143, 153, 163; 132, 142, 152, 162, 134, 144, 154, 164) erstreckt bzw. erstrecken.

21. Vorrichtung nach Anspruch 20, **dadurch gekennzeichnet, dass** sie, da die Mündungen der Leitungen (131, 141, 151, 161, 133, 143, 153, 163; 132, 142, 152, 162, 134, 144, 154, 164) über die beiden gegenüberliegenden Seitenwände (11, 12) des Behälters (100) verteilt sind, zwei Gitter (191, 192) umfasst, die sich gegenüber einer der Seitenwände (11, 12) erstrecken.

22. Vorrichtung nach einem der Ansprüche 15 bis 21, **dadurch gekennzeichnet, dass** das bzw. die Gitter aus Metall oder Plastik bestehen.

23. Verfahren zur Zellkultur (10), umfassend folgende Schritte:
- Anlegen der Zellen (10) zu einer Kultur in einem Behälter (100),
- Injizieren in den Behälter (100) während einer bestimmten Zeit einer ersten Fluidart (M1) über mindestens eine erste Injektionsleitung (131, 141, 151, 161, 132, 142, 152, 162),
- Ablassen der ersten Fluidart (M1) und gleichzeitiges Injizieren in den Behälter einer zweiten Fluidart (M2) über mindestens eine zweite Injektionsleitung (133, 143, 153, 163, 134, 144, 154, 164),
**dadurch gekennzeichnet, dass** während des Schritts des Injizierens der zweiten Fluidart (M2) das in der ersten Leitung (131, 141, 151, 161, 132, 142, 152, 162) enthaltene Fluid (M1) angesaugt wird, so dass sich das in der ersten Leitung (131, 141, 151, 161, 132, 142, 152, 162) enthaltene Fluid (M1) nicht in das Fluid (M2), das in den Behälter (100) injiziert wird, ausbreitet.

24. Verfahren nach Anspruch 23, **dadurch gekennzeichnet, dass** das in der ersten Leitung enthaltene Fluid (M1) über eine Rückinjektionsleitung (181, 182) angesaugt wird, deren eines Ende seitlich an die erste Injektionsleitung (131, 141, 151, 161, 133, 143, 153, 163) angeschlossen ist und deren anderes Ende mit Pumpmitteln verbunden ist.

25. Verfahren nach einem der Ansprüche 23 oder 24, **dadurch gekennzeichnet, dass** es ferner einen Schritt umfasst, bei dem die zweite Fluidart (M2) abgelassen wird, und die erste Fluidart (M1) erneut über die erste Injektionsleitung (131, 141, 151, 161, 132, 142, 152, 162) in den Behälter (100) injiziert wird, und dass die folgenden Schritte wiederholt werden, so dass die Zellen (10) nacheinander in verschiedenen Fluidarten liegen.

26. Verfahren nach einem der Ansprüche 23 bis 25, **dadurch gekennzeichnet, dass** während der Injektion eines der Fluide, die Zellen (10) einem elektrischen Feld ausgesetzt werden.

27. Verfahren nach einem der vorhergehenden Ansprüche, **dadurch gekennzeichnet, dass** der Schritt des Ersetzens eines Fluids durch ein anderes in dem Behälter ungefähr 1 Sekunde dauert.
